# EUROPEAN PATENT SPECIFICATION

(11) **EP 4 384 518 B1**
(45) Date of publication and mention of the grant of the patent: **17.12.2025**
(21) Application number: 22757992.7
(22) Date of filing: 10.08.2022
(51) Int. Cl.: C07D 487/04, A61P 35/00

(54) **TYROSINE KINASE INHIBITORS**
TYROSINKINASEHEMMER
INHIBITEURS DE LA TYROSINE KINASE

(30) Priority: 11.08.2021 GB 202111558
(43) Date of publication of application: 19.06.2024
(73) Proprietor: The University Court Of The University of Edinburgh, Edinburgh, Lothian EH8 9YL (GB)
(72) Inventor: TEMPS, Carolin, Edinburgh Lothian EH4 2XR (GB); CROKE, Stephen, Edinburgh Lothian EH4 2XR (GB); O. CARRAGHER, Neil, Edinburgh Lothian EH4 2XR (GB); UNCITI-BROCETA, Asier, Edinburgh Lothian EH4 2XR (GB)
(74) Representative: Murgitroyd & Company
(86) International application number: PCT/GB2022/052084
(87) International publication number: WO 2023/017264

(56) References cited:
- WO-A1-2016/185160
- CRAIG FRASER ET AL: "Rapid Discovery and Structure-Activity Relationships of Pyrazolopyrimidines That Potently Suppress Breast Cancer Cell Growth via SRC Kinase Inhibition with Exceptional Selectivity over ABL Kinase", JOURNAL OF MEDICINAL CHEMISTRY, AMERICAN CHEMICAL SOCIETY, US, vol. 59, no. 10, 26 May 2016 (2016-05-26), pages 4697 - 4710, XP009539157, ISSN: 0022-2623, [retrieved on 20150504], DOI: 10.1021/ACS.JMEDCHEM.6B00065

## Description

### BACKGROUND

The SRC protein and its family members are non-receptor tyrosine kinases that play a fundamental role in signal transduction pathways regulated by multiple cell surface receptors, thus controlling a wide range of cellular events including cell growth, survival, cell adhesion and migration. The oncogenic role of SRC in cancer was first proposed in the 1970's. Since then, accumulating evidence has demonstrated disease-associated upregulation of SRC kinase activity in many malignancies, including breast and colorectal cancer, and its direct involvement in cancer metastases and chemoresistance mechanisms. Despite the validated etiological role of this target and the number of commercial drug discovery programs developed across more than three decades, to date there are no kinase inhibitors approved to treat cancers by virtue of their SRC inhibitory properties alone. Interestingly, all ATP-competitive SRC kinase inhibitors either in the clinic (dasatanib, bosutinib) or under clinical development (saracatinib, AZD0424) are actually dual ABL/SRC inhibitors with additional off-target activities, as disclosed by L. N; Eadens, M; Messersmith, W. "Current status of SRC inhibitors in solid tumor malignancies", Oncologist, 2011, 16, 566-578; and by Roskoski, R. Jr. "Src protein-tyrosine kinase structure, mechanism, and small molecule inhibitors", Pharmacol. Res. 2015, 94, 9-25.

While lack of clinical efficacy rather than poor safety profile is the generally attributed cause for the failed entry of SRC inhibitors in the clinic, the off-target activities of current inhibitors reduce their therapeutic window. For example, the manifestation of cardiotoxic events is a well-established side effect of ABL inhibitors as shown for example by Steegmann, J. L. et al in "Off-target effects of BCR-ABL1 inhibitors and their potential long-term implications in patients with chronic myeloid leukemia", Leuk. Lymphoma 2012, 53, 2351-2361; and by Nygaard, H. B. et al in "A phase Ib multiple ascending dose study of the safety, tolerability, and central nervous system availability of AZD0530 (saracatinib) in Alzheimer's disease", Alzheimers Res. Ther. 2015, 7, 35.

In addition, several studies have shown that ABL plays an anti-oncogenic role in some cancers. For instance, ABL signaling exerts an anticancer effect during mammary tumorigenesis through the suppression of oncogenic TGF-β signaling (Allington, T. M. et al in "Activated Abl kinase inhibits oncogenic transforming growth factor-beta signaling and tumorigenesis in mammary tumors", FASEB J. 2009, 23, 4231-4243. Upon DNA damage, ABL is activated and contributes to apoptosis by an adhesion-dependent death pathway that is independent of p53 (Truong, T; Sun, G. et al in "Modulation of DNA damage-induced apoptosis by cell adhesion is independently mediated by p53 and c-Abl", Proc. Natl Acad. Sci. USA 2003, 100, 10281-10286). These studies strongly caution against the use of ABL kinase inhibitors in the treatment of some breast cancer subtypes and as combination partners with DNA damaging chemotherapeutic agents, particularly in metastatic tumours with mutant p53.

Another example of off-target activities that reduce drug tolerability of current inhibitors is their effect on the receptor tyrosine kinase KIT. Inhibition of KIT activity, a protein kinase that is targeted by dasatinib and bosutinib, lead to myelosupression, which can not only affect the immune defense of the patient against infectious diseases, but also reduce the capacity of the immune system to contribute in fighting cancer as described by Galanis et al. in Haematologica, 2015 Mar; 100(3): e77-e79. https://www.ncbi.nlm.nih.gov/pmc/articles/PMC4349281/.

Therefore, drugs that inhibit dysregulated SRC activity with high selectivity over specific kinases are sought after in cancer therapy because they would be devoid of side effects caused by promiscuous kinase inhibitors of SRC (dasatinib, bosutinib), e.g. the manifestation of toxicity events, immunosuppression, and pro-oncogenic effects.

WO 2016/185160 discloses tyrosine kinase inhibitors for use as a medicament. There is however still the need to identify other tyrosine kinase inhibitors having potent antiproliferative properties, which are safe to use and have suitable pharmacokinetics properties for treating different kinds of cancers and SRC-associated disorders. Furthermore, in addition to improvements in activity and selectivity, such other tyrosine kinase inhibitors may have improved liberation, absorption, distribution, metabolism, excretion and/or toxicity (LADMET) properties.

### SUMMARY

The present disclosure relates to pyrazolopyrimidine compounds that are capable of inhibiting, or selectively inhibiting one or more tyrosine kinases. The compounds find applications in the treatment of a variety of disorders, including proliferative disorders such as cancer, bone, lung, neurological, and viral disorders.

In one aspect, the disclosure relates to a compound of formula (I), or a pharmaceutically acceptable salt thereof, 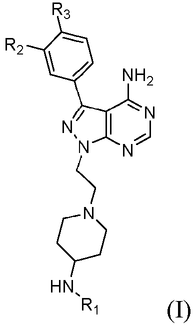 wherein:
R₁ is selected from a group consisting of alkyl, cycloalkyl, alkenyl, cycloalkenyl and alkynyl;
R₂ is selected from a group consisting of H, halo, OR₁₁, NHR₁₁, alkyl, alkenyl and alkynyl;
R₃ is selected from a group consisting of alkyl, alkenyl, alkynyl, aryl, halo, aryloxy, NHCO₂R₄, NHCONR₅R₆, NHCOR₇, NH-alkyl, NH-alkenyl, NH(CH₂)ₙ-aryl, (CH₂)ₚ-heteroaryl, (CH₂)_{q}CO₂R₈, (CH₂)ᵣCOR₉ and NHSO₂R₁₀, wherein each alkyl, alkenyl, aryl or heteroaryl moiety in the aforementioned list is optionally further substituted by one or more groups selected from alkyl, halo, OH, NH₂, alkoxy, aryloxy, alkylamino, arylamino, carboxyl and carboxamide;
R₄ to R₁₁ are each independently selected from a group consisting of alkyl, alkenyl and aryl; and
n is selected from 0, 1, 2, 3, 4, 5 and 6,
p is selected from 0, 1, 2, 3, 4, 5 and 6,
q is selected from 0, 1, 2, 3, 4, 5 and 6, and
r is selected from 0, 1, 2, 3, 4, 5 and 6.

In one aspect, the disclosure relates to a compound of formula (I), or a pharmaceutically acceptable salt thereof, 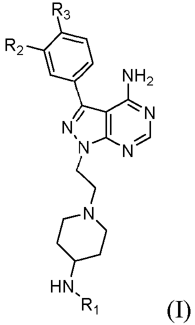 wherein:
R₁ is selected from a group consisting of C1₋₁₂-alkyl, C₃₋₈-cycloalkyl, C₂₋₁₂-alkenyl, C₃₋₈-cycloalkenyl and C₂₋₁₂-alkynyl;
R₂ is selected from a group consisting of H, halo, OR₁₁, NHR₁₁, C₁₋₁₂-alkyl, C₂₋₁₂-alkenyl and C₂₋₁₂-alkynyl;
R₃ is selected from a group consisting of C₁₋₁₂-alkyl, C₂₋₁₂-alkenyl, C₂₋₁₂-alkynyl, C₆₋₁₂-aryl, halo, C₆₋₁₂-aryloxy, NHCO₂R₄, NHCONR₅R₆, NHCOR₇, NH-C₁₋₁₂-alkyl, NH-C₂₋₁₂-alkenyl, NH(CH₂)ₙ-C₆₋₁₂-aryl, (CH₂)ₚ-heteroaryl, (CH₂)_{q}CO₂R₈, (CH₂)ᵣCOR₉ and NHSO₂R₁₀, wherein each alkyl, alkenyl, aryl or heteroaryl moiety in the aforementioned list is optionally further substituted by one or more groups selected from C₁₋₁₂-alkyl, halo, OH, NH₂, C₁₋₁₂-alkoxy, C₆₋₁₂-aryloxy, C₁₋₁₂-alkylamino, C₆₋₁₂-arylamino, carboxyl and carboxamide;
R₄ to R₁₁ are each independently selected from a group consisting of C₁₋₁₂-alkyl, C₂₋₁₂-alkenyl and C₆₋₁₂-aryl; and
n is selected from a group consisting of 0, 1, 2, 3, 4, 5 and 6,
p is selected from a group consisting of 0, 1, 2, 3, 4, 5 and 6,
q is selected from a group consisting of 0, 1, 2, 3, 4, 5 and 6, and
r is selected from a group consisting of 0, 1, 2, 3, 4, 5 and 6.

In one aspect, the disclosure relates to a compound of formula (I), or a pharmaceutically acceptable salt thereof, 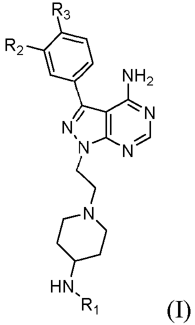 wherein:
R₁ is selected from a group consisting of C₁₋₄-alkyl, C₃₋₆-cycloalkyl, C₂₋₄-alkenyl, C₃₋₆-cycloalkenyl and C₂₋₄-alkynyl;
R₂ is selected from a group consisting of H, halo, OR₁₁, NHR₁₁, C₁₋₄-alkyl, C₂₋₄-alkenyl and C₂₋₄-alkynyl;
R₃ is selected from a group consisting of C₁₋₄-alkyl, C₂₋₄-alkenyl, C₂₋₄-alkynyl, C₆₋₁₂-aryl, halo, C₆₋₁₂-aryloxy, NHCO₂R₄, NHCONR₅R₆, NHCOR₇, NH-C₁₋₄-alkyl, NH-C₂₋₄-alkenyl, NH(CH₂)ₙ-C₆₋₁₂-aryl, (CH₂)ₚ-heteroaryl, (CH₂)_{q}CO₂R₈, (CH₂)ᵣCOR₉ and NHSO₂R₁₀, wherein each alkyl, alkenyl, aryl or heteroaryl moiety in the aforementioned list is optionally further substituted by one or more groups selected from C₁₋₄-alkyl, halo, OH, NH₂, C₁₋₄-alkoxy, C₆₋₁₂-aryloxy, C₁₋₄-alkylamino, C₆₋₁₂-arylamino, carboxyl and carboxamide;
R₄ to R₁₁ are each independently selected from a group consisting of C₁₋₄-alkyl, C₂₋₄-alkenyl and C₆₋₁₂-aryl; and
n is selected from a group consisting of 0, 1, 2, 3, 4, 5 and 6,
p is selected from a group consisting of 0, 1, 2, 3, 4, 5 and 6,
q is selected from a group consisting of 0, 1, 2, 3, 4, 5 and 6, and
r is selected from a group consisting of 0, 1, 2, 3, 4, 5 and 6.

In one aspect, the disclosure relates to a compound of formula (I), or a pharmaceutically acceptable salt thereof, 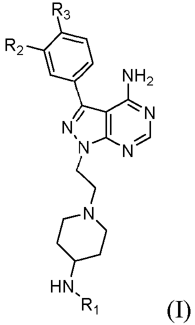 wherein:
R₁ is C₁₋₄-alkyl or C₂₋₄-alkenyl, preferably where it is CH₃ or CH(CH₃)₂;
R₂ is H or alkoxy, preferably where it is H or OCH₃;
R₃ is selected from a group consisting of NHCO₂-alkyl, NHCO-alkyl, NH(CH₂)ₙ-aryl, NHCONH-alkyl, (CH₂)ₚ-heteroaryl and (CH₂)_{q}CO₂-alkyl, preferably where it is selected from a group consisting of NHCO₂-^{t}Bu, NHCOCH₂C(CH₃)₃, NHCH₂phenyl, NHCONH-^{t}Bu, CH₂-(4-methyl-oxazol-2-yl) and CH₂CO₂- Bu;
R₄ to R₁₁ are each independently C₁₋₄-alkyl; and
n is selected from a group consisting of 0, 1, 2, 3, 4, 5 and 6,
p is selected from a group consisting of 0, 1, 2, 3, 4, 5 and 6,
q is selected from a group consisting of 0, 1, 2, 3, 4, 5 and 6, and
r is selected from a group consisting of 0, 1, 2, 3, 4, 5 and 6.

In one aspect, the disclosure relates to a compound of formula (I), where the structure is: 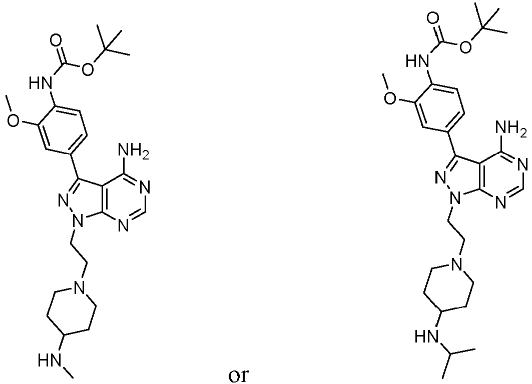 and pharmaceutically acceptable salts thereof.

In one aspect, the disclosure relates to a compound of formula (I) wherein the compound is in crystalline or amorphous form.

In one aspect, the disclosure relates to a pharmaceutical composition comprising at least one compound as described above and a pharmaceutically acceptable carrier, diluent or excipient.

In one aspect, the disclosure relates to a compound as described above for use in medicine.

In one aspect, the disclosure relates to a combination comprising a compound as described above and a second therapeutic agent.

In one aspect, the disclosure relates to a method of administering a compound of formula (I) to a mammal having a disease state alleviated by the selective inhibition of a SRC family kinase.

In one aspect, the disclosure relates to use of a compound of formula (I) in the manufacture of a medicament.

In a further aspect, the disclosure relates to a process for preparing compounds as described herein.

### BRIEF DESCRIPTION OF THE DRAWINGS

FIG. 1 depicts metabolite profiling for *tert*-butyl (4-(4-amino-1-(2-(4-(dimethylamino)piperidin-1-yl)ethyl)-1H-pyrazolo[3,4-d]pyrimidin-3-yl)-2-methoxyphenyl)carbamate (**506**).
FIG. 2 depicts dose response curves of compounds **506** and **5a** in breast cancer cells: MDA-MB-231 (n=3) and MDA-MB-436 (n=2).
FIG. 3 depicts dose response curves of compounds **506** and **5a** in ovarian cancer cells.
FIG. 4 shows a comparison between the GI50 values of compounds **506** and **5a** in ovarian cancer cells.
FIG. 5 shows ovarian cancer spheroid photos under treatement with DMSO (untreated control), **506, 5a** and dasatinib.
FIG. 6 depicts dose response curves of compounds **5a** and **8** against MDA-MB-231 breast cancer cells.

### DETAILED DESCRIPTION

The present disclosure relates to pyrazolopyrimidine compounds of formula (I) and pharmaceutically acceptable salts thereof that are capable of inhibiting the SRC kinase and its family members (LYN, FYN, LCK, HCK, FGR, BLK, FRK and YES). Preferebly, the compounds inhibit SRC and its family members with selectivity over other kinases, including the kinase ABL. In particular, the disclosure relates to substituted pyrazolopyrimidine compounds having a specific substitution pattern.

For example, provided herein is a compound of formula (I), or a pharmaceutically acceptable salt thereof, 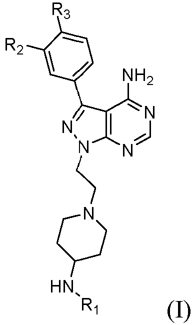 wherein:
R₁ is alkyl, cycloalkyl, alkenyl, cycloalkenyl or alkynyl;
R₂ is selected from H, halo, OR₁₁, NHR₁₁, alkyl, alkenyl and alkynyl;
R₃ is selected from alkyl, alkenyl, alkynyl, aryl, halo, aryloxy, NHCO₂R₄, NHCONR₅R₆, NHCOR₇, NH-alkyl, NH-alkenyl, NH(CH₂)ₙ-aryl, (CH₂)ₚ-heteroaryl, (CH₂)_{q}CO₂R₈, (CH₂)ᵣCOR₉ and NHSO₂R₁₀, wherein each alkyl, alkenyl, aryl or heteroaryl moiety in the aforementioned list is optionally further substituted by one or more groups selected from alkyl, halo, OH, NH₂, alkoxy, aryloxy, alkylamino, arylamino, carboxyl and carboxamide;
R₄ to R₁₁ are each independently selected from alkyl, alkenyl and aryl; and
n is selected from 0, 1, 2, 3, 4, 5 and 6,
p is selected from 0, 1, 2, 3, 4, 5 and 6,
q is selected from 0, 1, 2, 3, 4, 5 and 6, and
r is selected from 0, 1, 2, 3, 4, 5 and 6.

In some embodiments, wherein:
R₁ is C₁₋₁₂-alkyl, C₃₋₈-cycloalkyl, C₂₋₁₂-alkenyl, C₃₋₈-cycloalkenyl or C₂₋₁₂-alkynyl;
R₂ is selected from H, halo, OR₁₁, NHR₁₁, C₁₋₁₂-alkyl, C₂₋₁₂-alkenyl and C₂₋₁₂-alkynyl;
R₃ is selected from C₁₋₁₂-alkyl, C₂₋₁₂-alkenyl, C₂₋₁₂-alkynyl, C₆₋₁₂-aryl, halo, C₆₋₁₂-aryloxy, NHCO₂R₄, NHCONR₅R₆, NHCOR₇, NH-C₁₋₁₂-alkyl, NH-C₂₋₁₂-alkenyl, NH(CH₂)ₙ-C₆₋₁₂-aryl, (CH₂)ₚ-heteroaryl, (CH₂)_{q}CO₂R₈, (CH₂)ᵣCOR₉ and NHSO₂R₁₀, wherein each alkyl, alkenyl, aryl or heteroaryl moiety in the aforementioned list is optionally further substituted by one or more groups selected from C₁₋₁₂-alkyl, halo, OH, NH₂, C₁₋₁₂-alkoxy, C₆₋₁₂-aryloxy, C₁₋₁₂-alkylamino, C₆₋₁₂-arylamino, carboxyl and carboxamide;
R₄ to R₁₁ are each independently selected from C₁₋₁₂-alkyl, C₂₋₁₂-alkenyl and C₆₋₁₂-aryl; and
n is selected from 0, 1, 2, 3, 4, 5 and 6,
p is selected from 0, 1, 2, 3, 4, 5 and 6,
q is selected from 0, 1, 2, 3, 4, 5 and 6, and
r is selected from 0, 1, 2, 3, 4, 5 and 6.

In some embodiments, wherein:
R₁ is C₁₋₄-alkyl, C₃₋₆-cycloalkyl, C₂₋₄-alkenyl, C₃₋₆-cycloalkenyl or C₂₋₄-alkynyl;
R₂ is selected from H, halo, OR₁₁, NHR₁₁, C₁₋₄-alkyl, C₂₋₄-alkenyl and C₂₋₄-alkynyl;
R₃ is selected from C₁₋₄-alkyl, C₂₋₄-alkenyl, C₂₋₄-alkynyl, C₆₋₁₂-aryl, halo, C₆₋₁₂-aryloxy, NHCO₂R₄, NHCONR₅R₆, NHCOR₇, NH-C₁₋₄-alkyl, NH-C₂₋₄-alkenyl, NH(CH₂)ₙ-C₆₋₁₂-aryl, (CH₂)ₚ-heteroaryl, (CH₂)_{q}CO₂R₈, (CH₂)ᵣCOR₉ and NHSO₂R₁₀, wherein each alkyl, alkenyl, aryl or heteroaryl moiety in the aforementioned list is optionally further substituted by one or more groups selected from C₁₋₄-alkyl, halo, OH, NH₂, C₁₋₄-alkoxy, C₆₋₁₂-aryloxy, C₁₋₄-alkylamino, C₆₋₁₂-arylamino, carboxyl and carboxamide;
R₄ to R₁₁ are each independently selected from C₁₋₄-alkyl, C₂₋₄-alkenyl and C₆₋₁₂-aryl; and
n is selected from 0, 1, 2, 3, 4, 5 and 6,
p is selected from 0, 1, 2, 3, 4, 5 and 6,
q is selected from 0, 1, 2, 3, 4, 5 and 6, and
r is selected from 0, 1, 2, 3, 4, 5 and 6.

In one embodiment of the disclosure R₁ is alkyl or alkenyl.

According to one embodiment of the disclosure R₁ is CH₃. According to another embodiment, R₁ is CH(CH₃)₂.

According to another embodiment R₂ is selected from H and alkoxy, more preferably, H and OCH₃.

In one embodiment R₄ to R₁₁ are each independently alkyl.

According to a further embodiment R₃ is selected from NHCO₂-alkyl, NHCO-alkyl, NH(CH₂)ₙ-aryl, NHCONH-alkyl, (CH₂)ₚ-heteroaryl and (CH₂)_{q}CO₂-alkyl.

According to one embodiment R₃ is selected from NHCO₂-^{t}Bu, NHCOCH₂C(CH₃)₃, NHCH₂phenyl, NHCONH-^{t}Bu, CH₂-(4-methyl-oxazol-2-yl) and CH₂CO₂-^{t}Bu.

The disclosure also relates to a compound of formula (I) which structure is: 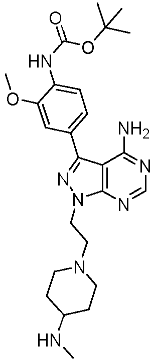 and pharmaceutically acceptable salts thereof.

The above compound is also hereafter referred as *tert*-butyl (4-(4-amino-1-(2-(4-(methylamino)piperidin-1-yl)ethyl)-1*H*-pyrazolo[3,4-d]pyrimidin-3-yl)-2-methoxyphenyl)carbamate (compound **5a**).

Further, the disclosure relates to a compound of formula (I) which structure is: 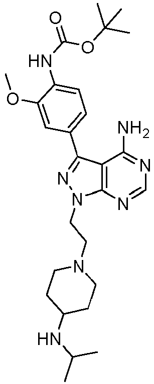 and pharmaceutically acceptable salts thereof.

The above compound is also hereafter referred as *tert*-butyl (4-(4-amino-1-(2-(4-(isopropylamino)piperidin-1-yl)ethyl)-1*H*-pyrazolo[3,4-d]pyrimidin-3-yl)-2-methoxyphenyl)carbamate (compound **8**).

In one aspect, the disclosure relates to a compound of formula (I) wherein the compound is in crystalline or amorphous form.

In one aspect, the disclosure relates to a pharmaceutical composition comprising at least one compound as described above and a pharmaceutically acceptable carrier, diluent or excipient.

In one aspect, the disclosure relates to a compound as described above for use in medicine.

The compound as described above may be used for treating a mammal having a disease state alleviated by the inhibition of a tyrosine kinase or the selective inhibition of a SRC family kinase.

The disclosure also relates to a compound as described above for use in treating cancer; or a viral infection; or osteoporosis; or a neurological disorder; or angiogenesis-dependent diseases; or a lung disease; or metastatic bone disease, or atherosclerosis or restenosis, e.g., characterized by migration and hyperproliferation of vascular smooth muscle cells.

The neurological disorder may be Alzheimer's disease.

The cancer may be breast cancer, colon cancer, prostate, melanoma, bladder, pancreatic, central nervous system (CNS), head and neck and ovarian cancer. The CNS cancer may be glioblastoma multiforme or medulloglastoma, e.g., medulloblastoma group 4.
In some embodiments the cancer is a cancer with metastasis. In some embodiments, the cancer is without metastasis.

In some embodiments the breast cancer is triple negative breast cancer, HER2 positive breast cancer, e.g., resistant to HER2 therapies, or estrogen positive, e.g., resistant to hormonal treatments and/or estrogen degraders such as tamoxifen or fulvestrant.

In some embodiments, the cancer is a cancer having a YES or SRC gene amplification.

In some embodiments the lung disease is - Interstitial lung disease, for example, idiopathic pulmonary fibrosis (IPF).

The references to methods of treatment in the subsequent paragraphs of this description are to be interpreted as references to the compounds, pharmaceutical compositions and medicaments of the present invention for use in a method for treatment of the human (or animal) body by therapy (or for diagnosis).

The disclosure also pertains to a method of treating a mammal having a disease selected from cancer, osteoporosis, neurological disorder, angiogenesis-dependent diseases, lung disease or metastatic bone disease, atherosclerosis or restenosis, e.g., characterized by migration and hyperproliferation of vascular smooth muscle cells, wherein the method comprises administering to a mammal a therapeutically effective amount of the compound as described above.

The neurological disorder may be Alzheimer's disease.

The cancer may be breast cancer, colon cancer, prostate, melanoma, bladder, pancreatic, central nervous system (CNS), head and neck and ovarian cancer. The CNS cancer may be glioblastoma multiforme or medulloglastoma, e.g., medulloblastoma group 4.

In some embodiments the cancer is a cancer with metastasis. In some embodiments, the cancer is without metastasis.

In some embodiments the breast cancer is triple negative breast cancer, HER2 positive breast cancer, e.g., resistant to HER2 therapies, or estrogen positive, e.g., resistant to hormonal treatments and/or estrogen degraders such as tamoxifen or fulvestrant.

In some embodiments, the cancer is a cancer having a YES or SRC gene amplification.

In some embodiments the lung disease is - Interstitial lung disease, for example, idiopathic pulmonary fibrosis (IPF).

In one aspect, of the disclosure relates to a combination comprising a compound as described above and a second therapeutic agent.

In one aspect, the disclosure relates to a method of administering a compound of formula (I) to a mammal having a disease state alleviated by the selective inhibition of a SRC family kinase.

In one aspect, the disclosure relates to a method of administering a compound of formula (I) to a mammal having a disease state selected from cancer, osteoporosis, a neurological disorder, angiogenesis-dependent diseases, lung disease or metastatic bone disease, atherosclerosis or restenosis (e.g., characterized by migration and hyperproliferation of vascular smooth muscle cells), wherein the method comprises administering to a mammal a therapeutically effective amount of the compound.

The neurological disorder may be Alzheimer's disease.

The cancer may be breast cancer, colon cancer, prostate, melanoma, bladder, pancreatic, central nervous system (CNS), head and neck and ovarian cancer. The CNS cancer may be glioblastoma multiforme or medulloglastoma, e.g., medulloblastoma group 4.
In some embodiments the cancer is a cancer with metastasis. In some embodiments, the cancer is without metastasis.

In some embodiments the breast cancer is triple negative breast cancer, HER2 positive breast cancer, e.g., resistant to HER2 therapies, or estrogen positive, e.g., resistant to hormonal treatments and/or estrogen degraders such as tamoxifen or fulvestrant.

In some embodiments, the cancer is a cancer having a YES or SRC gene amplification.

In some embodiments the lung disease is - Interstitial lung disease, for example, idiopathic pulmonary fibrosis (IPF).

In one aspect, the disclosure relates to use of a compound of formula (I) in the manufacture of a medicament.

In one aspect, the disclosure relates to use of a compound of formula (I) in the manufacture of a medicament for the treatment of a mammal having a disease state alleviated by the selective inhibition of a SRC family kinase.

In one aspect, the disclosure relates to use of a compound of formula (I) in the manufacture of a medicament for the treatment of cancer, osteoporosis, a neurological disorder, angiogenesis-dependent diseases, lung disease or metastatic bone disease, atherosclerosis or restenosis e.g., characterized by migration and hyperproliferation of vascular smooth muscle cells.

The neurological disorder may be Alzheimer's disease.

The cancer may be breast cancer, colon cancer, prostate, melanoma, bladder, pancreatic, central nervous system (CNS), head and neck and ovarian cancer. The CNS cancer may be glioblastoma multiforme or medulloglastoma, e.g., medulloblastoma group 4.

In some embodiments the cancer is a cancer with metastasis. In some embodiments, the cancer is without metastasis.

In some embodiments the breast cancer is triple negative breast cancer, HER2 positive breast cancer, e.g., resistant to HER2 therapies, or estrogen positive, e.g., resistant to hormonal treatments and/or estrogen degraders such as tamoxifen or fulvestrant.

In some embodiments, the cancer is a cancer having a YES or SRC gene amplification.

In some embodiments the lung disease is - Interstitial lung disease, for example, idiopathic pulmonary fibrosis (IPF).

In a further aspect, the invention relates to a process for preparing compounds as described herein.

"Alkyl" is defined herein as a straight-chain or branched alkyl radical, for example, methyl, ethyl, propyl, isopropyl, butyl, isobutyl, tert-butyl, pentyl, hexyl. Preferably, the alkyl group is a C₁₋₁₂-alkyl group, more preferably, a C₁₋₆-alkyl group, even more preferably a C₁₋₄-alkyl group.

"Alkenyl" is defined herein as a straight-chain or branched radical, containing one or more carbon-carbon double bonds. Preferably, the alkenyl group is a C₂₋₁₂-alkyl group, more preferably, a C₂₋₆-alkyl group, even more preferably a C₂₋₄-alkyl group.

"Alkynyl" is defined herein as a straight-chain or branched radical, containing one or more carbon-carbon triple bonds. Preferably, the alkynyl group is a C₂₋₁₂-alkyl group, more preferably, a C₂₋₆-alkynyl group, even more preferably a C₂₋₄-alkynyl group.

"Cycloalkyl" is defined herein as a monocyclic alkyl ring, such as, cyclopropyl, cyclobutyl, cyclopentyl, cyclohexyl or cycloheptyl, or a fused bicyclic ring system such as norbornane. Preferably, the cycloalkyl group is a C₃₋₈-cycloalkyl group, more preferably a C₃₋₆-cycloalkyl group.

"Cycloalkenyl" is defined herein as a cyclic group as defined above for cycloalkyl, but containing one or more carbon-carbon double bonds. Preferably, the cycloalkenyl group is a C₃₋₈-cycloalkenyl group, more preferably a C₃₋₆-cycloalkenyl group.

As used herein, the term "aryl" refers to a C₆₋₁₂ aromatic group, which may be benzocondensed, for example, phenyl or naphthyl.

"Alkoxy" is a group wherein an alkyl as defined above is singularly bonded to oxygen.

"Aryloxy" is a group wherein an aryl as defined above is singularly bonded to oxygen.

"Alkylamino" is a group wherein the alkyl as defined above is attached to the remainder of a molecule via nitrogen.

"Arylamino" is a group wherein the aryl as defined above is attached to the remainder of a molecule via nitrogen.

"Carboxamide" An amide of a carboxylic acid, having the structure RC(=O)NR₂. The term is used as a suffix in systematic name formation to denote the - C(=O)NH₂ group including its carbon atom.

"Halogen" or "halo" is defined herein as chloro, fluoro, bromo or iodo.

As used herein, the term "aryl" refers to a C₄₋₁₂ aromatic group, which may be benzocondensed, for example, phenyl or naphthyl.

"Heteroaryl" is defined herein as a monocyclic or bicyclic C₄₋₁₂ aromatic ring comprising one or more heteroatoms (that may be the same or different), such as oxygen, nitrogen or sulphur. Examples of suitable heteroaryl groups include thienyl, furanyl, pyrrolyl, pyridinyl, oxazolyl, thiazolyl, imidazolyl, pyrazolyl, isoxazolyl, isothiazolyl, oxadiazolyl, triazolyl, thiadiazolyl etc. and benzo derivatives thereof, such as benzofuranyl, benzothienyl, benzimidazolyl, indolyl, isoindolyl, indazolyl etc; or pyridyl, pyrazinyl, pyrimidinyl, pyridazinyl, triazinyl etc. and benzo derivatives thereof, such as quinolinyl, isoquinolinyl, cinnolinyl, phthalazinyl, quinazolinyl, quinoxalinyl, naphthyridinyl etc.

Any of these groups may be attached to the rest of the molecule at any suitable position.

The compounds of the disclosure can be present as salts or esters, in particular pharmaceutically and veterinarily acceptable salts or esters.

Pharmaceutically acceptable salts of the compounds of the disclosure include suitable acid addition or base salts thereof. A review of suitable pharmaceutical salts may be found in Berge et al, J Pharm Sci, 66, 1-19 (1977). Salts are formed, for example with strong inorganic acids such as mineral acids, e.g. hydrohalic acids such as hydrochloride, hydrobromide and hydroiodide, sulphuric acid, phosphoric acid sulphate, bisulphate, hemisulphate, thiocyanate, persulphate and sulphonic acids; with strong organic carboxylic acids, such as alkanecarboxylic acids of 1 to 4 carbon atoms which are unsubstituted or substituted (e.g., by halogen), such as acetic acid; with saturated or unsaturated dicarboxylic acids, for example oxalic, malonic, succinic, maleic, fumaric, phthalic or tetraphthalic; with hydroxycarboxylic acids, for example ascorbic, glycolic, lactic, malic, tartaric or citric acid; with aminoacids, for example aspartic or glutamic acid; with benzoic acid; or with organic sulfonic acids, such as (C₁-C₄)-alkyl- or aryl-sulfonic acids which are unsubstituted or substituted (for example, by a halogen) such as methane- or p-toluene sulfonic acid. Salts which are not pharmaceutically or veterinarily acceptable may still be valuable as intermediates.

Preferred salts include, for example, acetate, trifluoroacetate, lactate, gluconate, citrate, tartrate, maleate, malate, pantothenate, adipate, alginate, aspartate, benzoate, butyrate, digluconate, cyclopentanate, glucoheptanate, glycerophosphate, oxalate, heptanoate, hexanoate, fumarate, nicotinate, palmoate, pectinate, 3-phenylpropionate, picrate, pivalate, proprionate, tartrate, lactobionate, pivolate, camphorate, undecanoate and succinate, organic sulphonic acids such as methanesulphonate, ethanesulphonate, 2-hydroxyethane sulphonate, camphorsulphonate, 2-naphthalenesulphonate, benzenesulphonate, p-chlorobenzenesulphonate and p-toluenesulphonate; and inorganic acids such as hydrochloride, hydrobromide, hydroiodide, sulphate, bisulphate, hemisulphate, thiocyanate, persulphate, phosphoric and sulphonic acids.

The disclosure also includes, where appropriate all enantiomers, diastereoisomers and tautomers of the compounds of the disclosure. The person skilled in the art will recognise compounds that possess optical properties (one or more chiral carbon atoms) or tautomeric characteristics. The corresponding enantiomers and/or tautomers may be isolated/prepared by methods known in the art.

Enantiomers are characterised by the absolute configuration of their chiral centres and described by the R- and S-sequencing rules of Cahn, Ingold and Prelog. Such conventions are well known in the art (e.g. see 'Advanced Organic Chemistry', 3rd edition, ed. March, J., John Wiley and Sons, New York, 1985).

Compounds of the disclosure containing a chiral centre may be used as a racemic mixture, an enantiomerically enriched mixture, or the racemic mixture may be separated using well-known techniques and an individual enantiomer may be used alone.

Some of the compounds of the disclosure may exist as stereoisomers and/or geometric isomers - e.g. they may possess one or more asymmetric and/or geometric centres and so may exist in two or more stereoisomeric and/or geometric forms. The present disclosure contemplates the use of all the individual stereoisomers and geometric isomers of those inhibitor agents, and mixtures thereof. The terms used in the claims encompass these forms, provided said forms retain the appropriate functional activity (though not necessarily to the same degree).

The present disclosure also includes all suitable isotopic variations of the agent or a pharmaceutically acceptable salt thereof. An isotopic variation of an agent of the present disclosure or a pharmaceutically acceptable salt thereof is defined as one in which at least one atom is replaced by an atom having the same atomic number but an atomic mass different from the atomic mass usually found in nature. Examples of isotopes that can be incorporated into the agent and pharmaceutically acceptable salts thereof include isotopes of hydrogen, carbon, nitrogen, oxygen, phosphorus, sulphur, fluorine and chlorine such as ²H, ³H, ¹³C, ¹⁴C, ¹⁵N, ¹⁷O, ¹⁸O, ³¹P, ³²P, ³⁵S, ¹⁸F and ³⁶Cl, respectively. Certain isotopic variations of the agent and pharmaceutically acceptable salts thereof, for example, those in which a radioactive isotope such as ³H or ¹⁴C is incorporated, are useful in drug and/or substrate tissue distribution studies. Tritiated, i.e., ³H, and carbon-14, i.e., ¹⁴C, isotopes are particularly preferred for their ease of preparation and detectability. Further, substitution with isotopes such as deuterium, i.e., ²H, may afford certain therapeutic advantages resulting from greater metabolic stability, for example, increased *in vivo* half-life or reduced dosage requirements and hence may be preferred in some circumstances. For example, the disclosure includes compounds of general formula (I) where any hydrogen atom has been replaced by a deuterium atom. Isotopic variations of the agent of the present disclosure and pharmaceutically acceptable salts thereof of this disclosure can generally be prepared by conventional procedures using appropriate isotopic variations of suitable reagents.

The disclosure (not according to the invention) further includes the compounds of the present disclosure in prodrug form, i.e. covalently bonded compounds which release the active parent drug according to general formula (I) *in vivo.* Such prodrugs are generally compounds of the disclosure wherein one or more appropriate groups have been modified such that the modification may be reversed upon administration to a human or mammalian subject. This chemical modifications can be incorporated, for example, on the NH group connected to the piperidine group of compounds of formula (I). Modifications include generation of an amide or carbamate group. Reversion is usually performed by an enzyme naturally present in such subject, though it is possible for a second agent to be administered together with such a prodrug in order to perform the reversion *in vivo.* Examples of such modifications include amide or carbamate (for example, any of those described above), wherein the reversion may be carried out be an amidocarboxylase, etc. Other such systems will be well known to those skilled in the art.

The present disclosure also includes solvate forms of the compounds of the present disclosure. The terms used in the claims encompass these forms.

The disclosure further relates to the compounds of the present disclosure in their various crystalline forms, polymorphic forms and (an)hydrous forms. It is well established within the pharmaceutical industry that chemical compounds may be isolated in any of such forms by slightly varying the method of purification and or isolation form the solvents used in the synthetic preparation of such compounds.

The pharmaceutical compositions of the present disclosure may be adapted for rectal, nasal, intrabronchial, topical (including buccal and sublingual), vaginal or parenteral (including subcutaneous, intramuscular, intravenous, intraarterial and intradermal), intraperitoneal or intrathecal administration. Preferably the formulation is an orally administered formulation. The formulations may conveniently be presented in unit dosage form, i.e., in the form of discrete portions containing a unit dose, or a multiple or sub-unit of a unit dose. By way of example, the formulations may be in the form of tablets and sustained release capsules, and may be prepared by any method well known in the art of pharmacy.

Formulations for oral administration in the present disclosure may be presented as: discrete units such as capsules, gellules, drops, cachets, pills or tablets each containing a predetermined amount of the active agent; as a powder or granules; as a solution, emulsion or a suspension of the active agent in an aqueous liquid or a non-aqueous liquid; or as an oil-in-water liquid emulsion or a water-in-oil liquid emulsion; or as a bolus etc. Preferably, these compositions contain from 1 to 250 mg and more preferably from 10-100 mg, of active ingredient per dose.

For compositions for oral administration (e.g. tablets and capsules), the term "acceptable carrier" includes vehicles such as common excipients e.g. binding agents, for example syrup, acacia, gelatin, sorbitol, tragacanth, polyvinylpyrrolidone (Povidone), methylcellulose, ethylcellulose, sodium carboxymethylcellulose, hydroxypropylmethylcellulose, sucrose and starch; fillers and carriers, for example corn starch, gelatin, lactose, sucrose, microcrystalline cellulose, kaolin, mannitol, dicalcium phosphate, sodium chloride and alginic acid; and lubricants such as magnesium stearate, sodium stearate and other metallic stearates, glycerol stearate stearic acid, silicone fluid, talc waxes, oils and colloidal silica. Flavouring agents such as peppermint, oil of wintergreen, cherry flavouring and the like can also be used. It may be desirable to add a colouring agent to make the dosage form readily identifiable. Tablets may also be coated by methods well known in the art.

A tablet may be made by compression or moulding, optionally with one or more accessory ingredients. Compressed tablets may be prepared by compressing in a suitable machine the active agent in a free flowing form such as a powder or granules, optionally mixed with a binder, lubricant, inert diluent, preservative, surface-active or dispersing agent. Moulded tablets may be made by moulding in a suitable machine a mixture of the powdered compound moistened with an inert liquid diluent. The tablets may be optionally be coated or scored and may be formulated so as to provide slow or controlled release of the active agent.

Other formulations suitable for oral administration include lozenges comprising the active agent in a flavoured base, usually sucrose and acacia or tragacanth; pastilles comprising the active agent in an inert base such as gelatin and glycerin, or sucrose and acacia; and mouthwashes comprising the active agent in a suitable liquid carrier.

Other forms of administration comprise solutions or emulsions which may be injected intravenously, intraarterially, intrathecally, subcutaneously, intradermally, intraperitoneally or intramuscularly, and which are prepared from sterile or sterilisable solutions. Injectable forms typically contain between 10 - 1000 mg, preferably between 10 - 250 mg, of active ingredient per dose.

The pharmaceutical compositions of the present disclosure may also be in form of suppositories, pessaries, suspensions, emulsions, lotions, ointments, creams, gels, sprays, solutions or dusting powders.

An alternative means of transdermal administration is by use of a skin patch. For example, the active ingredient can be incorporated into a cream consisting of an aqueous emulsion of polyethylene glycols or liquid paraffin. The active ingredient can also be incorporated, at a concentration of between 1 and 10% by weight, into an ointment consisting of a white wax or white soft paraffin base together with such stabilisers and preservatives as may be required.

A person of ordinary skill in the art can easily determine an appropriate dose of one of the instant compositions to administer to a subject without undue experimentation. Typically, a physician will determine the actual dosage which will be most suitable for an individual patient and it will depend on a variety of factors including the activity of the specific compound employed, the metabolic stability and length of action of that compound, the age, body weight, general health, sex, diet, mode and time of administration, rate of excretion, drug combination, the severity of the particular condition, and the individual undergoing therapy. The dosages disclosed herein are exemplary of the average case. There can of course be individual instances where higher or lower dosage ranges are merited, and such are within the scope of this disclosure.

In accordance with this disclosure, an effective amount of a compound of general formula (I) may be administered to inhibit the kinase implicated with a particular condition or disease. Of course, this dosage amount will further be modified according to the type of administration of the compound. For example, to achieve an "effective amount" for acute therapy, parenteral administration of a compound of general formula (I) is preferred. An intravenous infusion of the compound in 5% dextrose in water or normal saline, or a similar formulation with suitable excipients, is most effective, although an intramuscular bolus injection is also useful. Typically, the parenteral dose will be about 0.01 to about 100 mg/kg; preferably between 0.1 and 20 mg/kg, in a manner to maintain the concentration of drug in the plasma at a concentration effective to inhibit a kinase. The compounds may be administered one to four times daily at a level to achieve a total daily dose of about 0.4 to about 400 mg/kg/day. The precise amount of an inventive compound which is therapeutically effective, and the route by which such compound is best administered, is readily determined by one of ordinary skill in the art by comparing the blood level of the agent to the concentration required to have a therapeutic effect.

The compounds of this disclosure may also be administered orally to the patient, in a manner such that the concentration of drug is sufficient to achieve one or more of the therapeutic indications disclosed herein. Typically, a pharmaceutical composition containing the compound is administered at an oral dose of between about 0.1 to about 100 mg/kg in a manner consistent with the condition of the patient. Preferably the oral dose would be about 0.5 to about 20 mg/kg.

No unacceptable toxicological effects are expected when compounds of the present disclosure are administered in accordance with the present disclosure. The compounds of this disclosure, which may have good bioavailability, may be tested in one of several biological assays to determine the concentration of a compound which is required to have a given pharmacological effect.

The one or more compounds of the disclosure may be administered in combination with one or more other active agents, for example, existing drugs available on the market. In such cases, the compounds of the disclosure may be administered consecutively, simultaneously or sequentially with the one or more other active agents.

Drugs in general may be more effective when used in combination. In particular, combination therapy is desirable in order to avoid an overlap of major toxicities, mechanism of action and resistance mechanism(s). Furthermore, it is also desirable to administer most drugs at their maximum tolerated doses with minimum time intervals between such doses. The major advantages of combining chemotherapeutic drugs are that it may promote additive or possible synergistic effects through biochemical interactions and also may decrease or delay the emergence of resistance.

Beneficial combinations may be suggested by studying the inhibitory activity of the test compounds with agents known or suspected of being valuable in the treatment of a particular disorder. For example, the disclosure relates to the use of a compound as described above in an assay for identifying compounds that promote additive and synergistic activity upon anti-cancer activities when combined with the compound. Preferably the assay is a high-throughput cell based phenotypic screen. This procedure can also be used to determine the order of administration of the agents, i.e. before, simultaneously, or after delivery. Such scheduling may be a feature of all the active agents identified herein.

One aspect of the disclosure relates to a process for preparing a compound of formula (I) as defined herein, said process comprising the steps of: 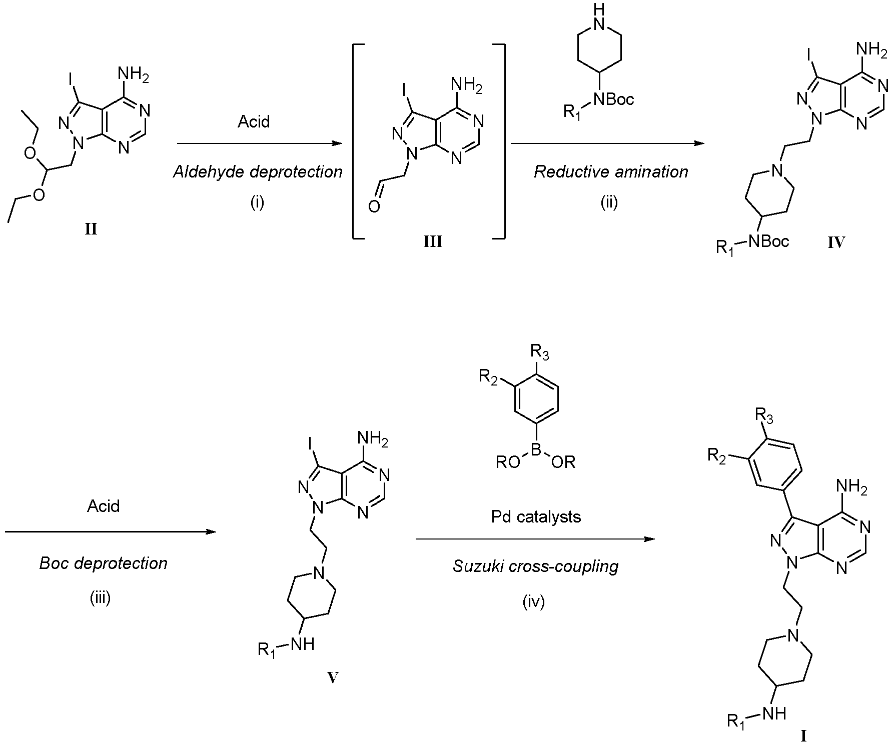
(i) converting a compound of formula (II) to a compound of formula (III);
(ii) converting said compound of formula (III) to a compound of formula (IV);
(iii) converting said compound of formula (IV) to a compound of formula (V); and
(iv) converting said compound of formula (V) to a compound of formula (I).

Preferred reagents for steps (i) to (iv) are: (i) TFA:H₂O or acetic acid, (ii) sodium triacetoxyborohydride; (iii) 4 N HCl in dioxane; (iv) K₂CO₃, PPh₃, Pd(OAc)₂ in dioxane:water (9:1).

### EXAMPLES

### Example 1: Compound 506 Metabolite Profiling

*Tert*-butyl (4-(4-amino-1-(2-(4-(dimethylamino)piperidin-1-yl)ethyl)-1H-pyrazolo[3,4-d]pyrimidin-3-yl)-2-methoxyphenyl)carbamate (compound **506**) is disclosed in WO2016/185160 and has the following structure:

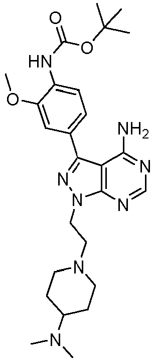

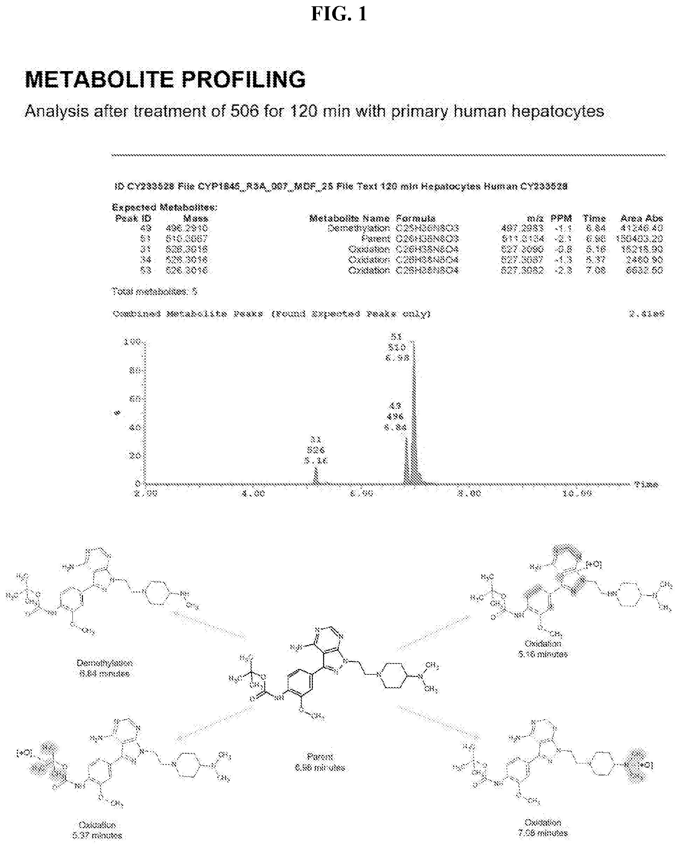

A metabolite profiling was performed in primary human hepatocytes and analysed using a Q-Tof UPLC-MS/MS platform. Hepatocytes were incubated in the presence of **506** at normal cell culture conditions for 60 min and 120 min, and samples analysed before and after treatment using an AB Sciex TripleTOF^{®} 6600. Profiling data in combination with fragmentation patterns were used to quantify and identify the parent compound and the resulting metabolites. Results from the study are shown in FIG. 1.

One of the metabolites found from this study (demethylated derivative: 10.5%, after 60 min; 19% after 120 min), *tert*-butyl (4-(4-amino-1-(2-(4-(methylamino)piperidin-1-yl)ethyl)-1H-pyrazolo[3,4-d]pyrimidin-3-yl)-2-methoxyphenyl)carbamate (**5a**) was synthesized in the lab. Surprisingly, screening in cancer cell lines showed that **5a** retains the potency and selectivity characteristics of the parent compound **506** (see data below).

### Example 2: Biological Activity Assays

### Methods & Materials

Cells were grown in Dulbecco's Modified Eagle Medium (DMEM) or Roswell Park Memorial Institute (RPMI) medium supplemented with serum (10% fetal bovine serum) and L-glutamine (2 mM) and incubated in a Heracell 240i tissue culture incubator at 37 °C and 5 % CO₂.

### Cell Viability Assay

Cells (breast cancer cells MDA-MB-231 and MDA-MB-436, and ovarian cancer cells ES-2, IGROV-1, OVCAR3, OVMANA, OVTOKO and TOV-21G) were plated in 96-well plates at 2000 cells/well in 100 µl of DMEM or RPMI medium containing 10% FBS and 2 mM L-glutamine and incubated for 48 h in an incubator at 37 °C and 5% CO₂. After 48 hours, the media was aspirated from each well and replaced with 95 µl of fresh medium. Compounds, including DMSO, were prepared at 20X in DMEM medium in a separate 96-well intermediate plate. 5 µl from the intermediate plate was then added to each well containing cells. Untreated cells were incubated with DMSO (0.1% v/v). After 5 days, PrestoBlue^{™} cell viability reagent (10 µl) was added to each well and the plates incubated for 60 - 90 min. Fluorescence emission was detected using a fluorescence plate reader (excitation 540 nm, emission 590 nm). All conditions were normalised to the untreated cells (100%) and dose-response curves were fitted using GraphPad Prism software.

### Spheroids Assays

Ovarian cancer cells (IGROV-1, OVCAR3, OVMANA, OVTOKO and TOV-21G, kindly donated by Prof Gourley's lab) were seeded on day 0 in ultra-low adhesion round bottom 96-well plates (Costar, 7007) at 2,000 cells per well in 200 µL of medium. Plates were centrifuged (1,000 g, 10 min, 4 °C, acceleration/deceleration 1/10) and incubated for 2 d to allow spheroids to form. Compounds were added to the wells without changing the medium (final DMSO concentration 0.1 % v/v) and the plates were incubated for 7 d. Spheroids were imaged on the day of treatment, 4 d post treatment and 7 d post treatment using the ImageXpress MicroXI platform. Eight Z-stack images (40 X, brightfield) were taken with 50 µM size step and collapsed into a single image from which the size of the spheroid was measured using CellProlifer. The size of each spheroid was calculated for all time points and expressed as percentage of spheroid size on the day of treatment start.

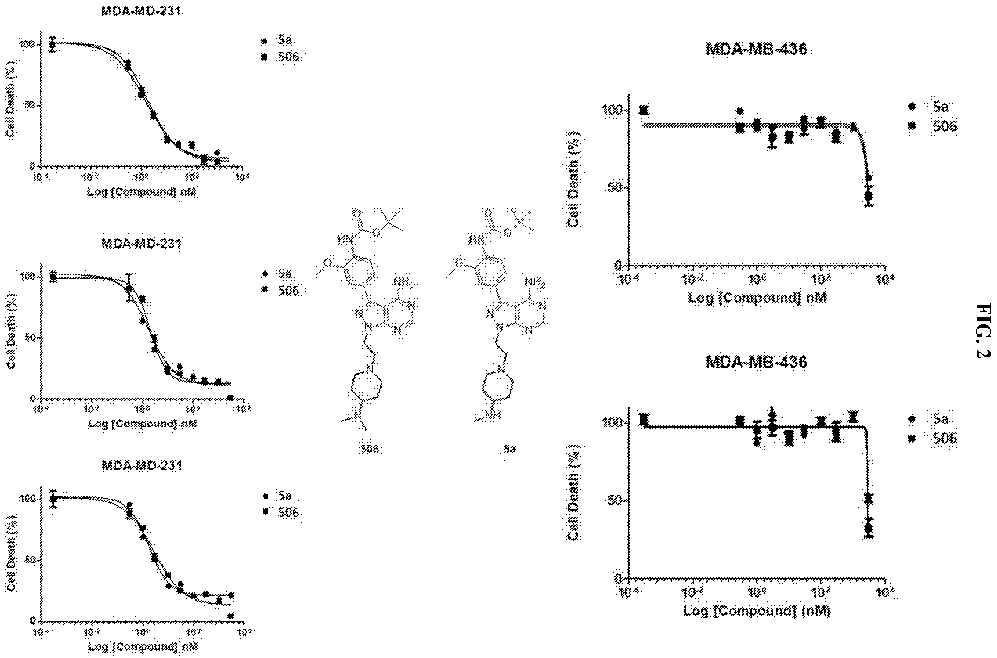

FIG. 2 shows dose response curves of **506** and **5a** in breast cancer cells: MDA-MB-231 (n=3) and MDA-MB-436 (n=2).

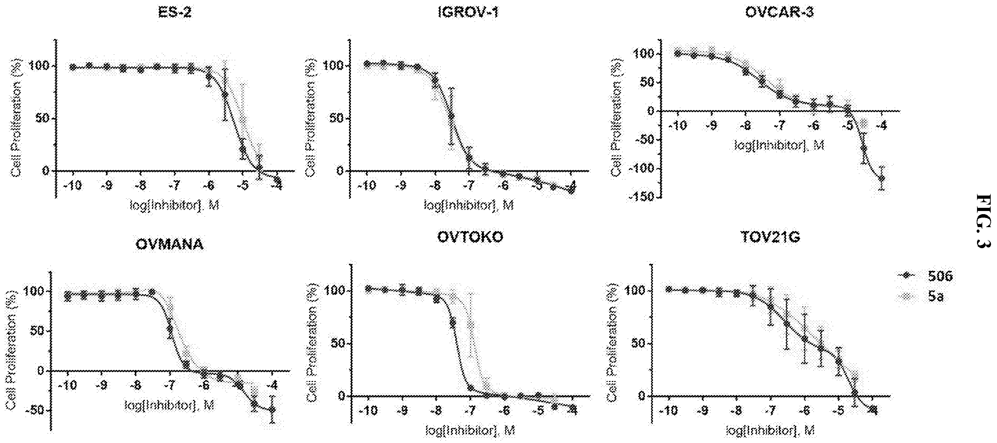

FIG. 3 shows dosee response curves of **506** and **5a** in ovarian cancer cells.

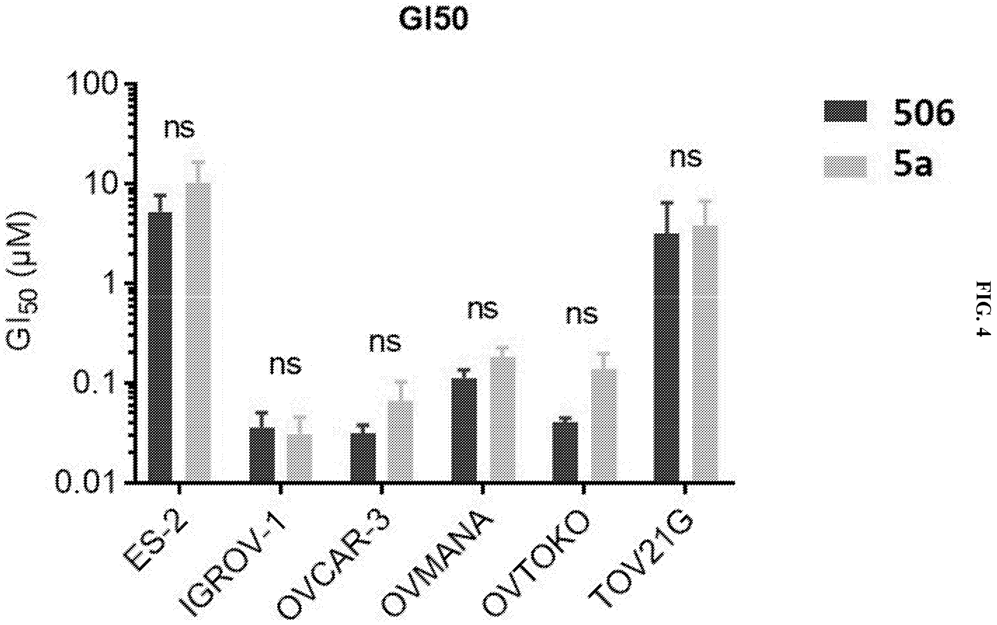

FIG. 4 shows a comparison between the GI50 values of **506** and **5a** in ovarian cancer cells.

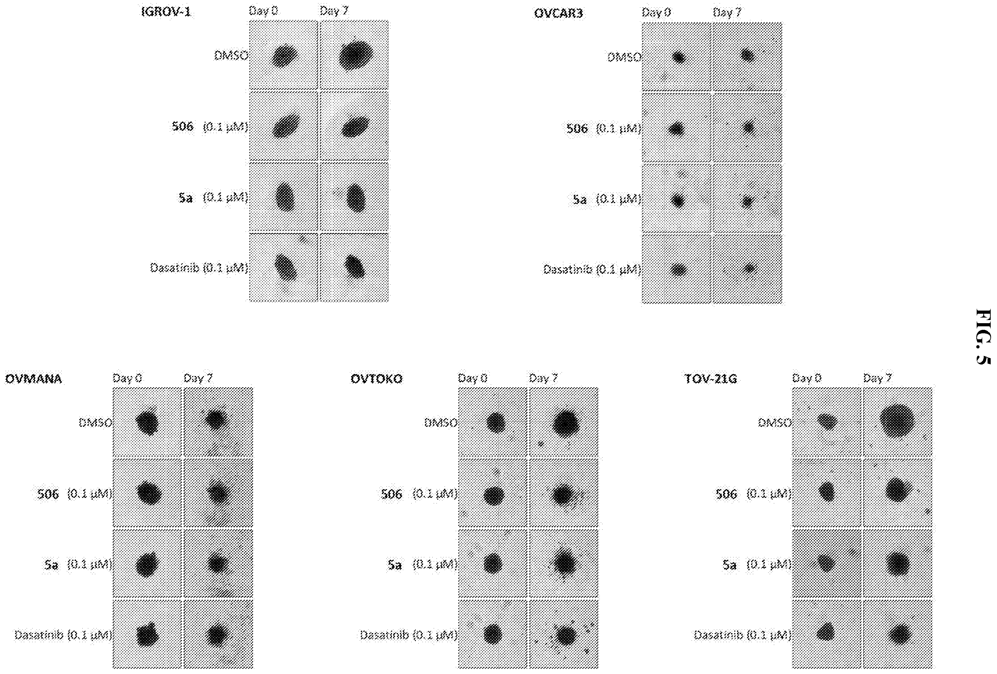

FIG. 5 shows ovarian cancer spheroid photos under treatement with DMSO (untreated control), **506, 5a** and dasatinib.

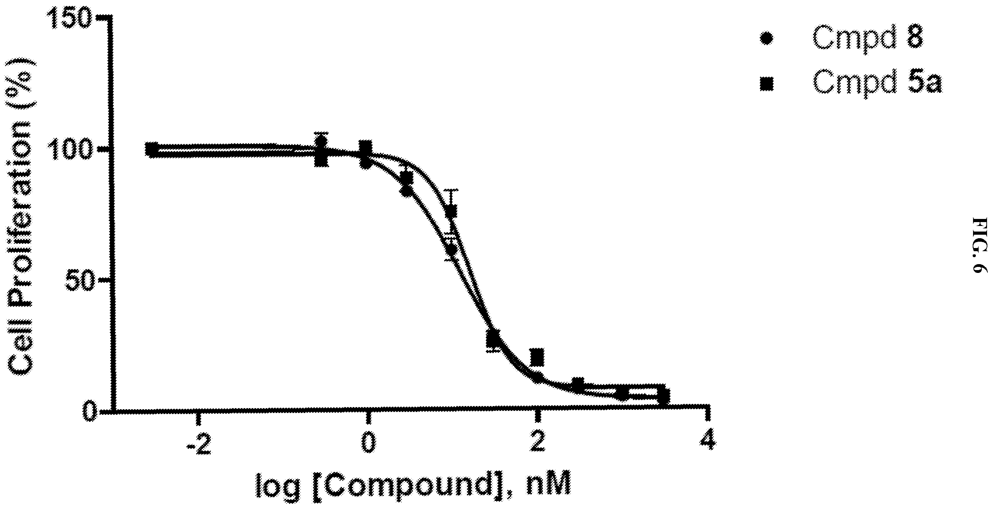

FIG. 6 depicts dose response curves of compounds **5a** and **8** against MDA-MB-231 breast cancer cells. Compound **5a** displayed a GI50 of 16.23 nM. Compound **8** displayed a GI50 of 12.48 nM.

### Kinase Screening Assay

Compounds 506, 5a and 8 were dissolved in dimethylsulfoxide (DMSO), and further diluted with assay buffer to a final concentration of 0.5 µmol/L. Staurosporine was positive control. A 4x Substrate/ATP/Metal (1 µmol/mL substrate, 5 µmol/mL ATP, 5 mmol/L Mg²⁺) solution was prepared with buffer (20 mmol/L HEPES, 0.01% Triton X-100, 5 mmol/L DTT, pH 7.5), and a 2x kinase solution was also prepared with buffer (20 mmol/L HEPES, 0.01% Triton X-100, 1 mmol/L DTT, pH 7.5). 5 µL of 4x compound solution, 5 µL of 4x Substrate/ATP/Metal solution, and 10 µL of 2x kinase solution were mixed and incubated in a well of polypropylene 384 well microplate for 1 hour at room temperature. 70 µL of Termination Buffer (QuickScout Screening Assist MSA) was added to the well. The reaction mixture was applied to LabChipTM system, and the product and substrate peptide peaks were separated and quantitated. The kinase reaction was evaluated by the product ratio calculated from peak heights of product(P) and substrate(S) peptides (P/(P+S)). The readout value of reaction control (complete reaction mixture) was set as a 0% inhibition, and the readout value of background (Enzyme(-)) was set as a 100% inhibition, then the percent inhibition of each test solution was calculated (Table 1).

**Table 1. Kinase screening (% inhibition at 0.5 micromolar)**

| **Kinase** | **Compound 506** | **Compound 5a** | **Compound 8** |
|---|---|---|---|
| ABL | 32.2 | 29.2 | 24.2 |
| BRK | 97.4 | 98.5 | 99.5 |
| EGFR | 46.1 | 46.3 | 58.2 |
| Her4 | 96.7 | 97.6 | 94.9 |
| KDR | 2.9 | 1.7 | -7.3 |
| KIT | 4.4 | 2.8 | 2.7 |
| PDGFR alpha | 13.5 | 13.6 | 12.2 |
| RET | 3.8 | -0.3 | -2.3 |
| SRC | 101.2 | 101.8 | 97.3 |
| FGR | 100.1 | 100.8 | 100.4 |
| FYNa | 100.5 | 99.4 | 100.2 |
| HCK | 101.3 | 104.6 | 98.7 |
| LCK | 104.0 | 106.9 | 101.3 |
| LYNa | 100.7 | 101.6 | 101.3 |
| YES | 99.7 | 99.7 | 101.9 |

Thus, it can be seen that compounds 5a and 8 inhibit SRC and its family members with selectivity over other kinases, and are more selective than compound 506.

### Example 3: Synthesis of Compounds

### Chromatography

Column chromatography refers to silica gel chromatography and was carried out manually using conventional glass columns and silica gel (pore size 60Å, particle size 230-400 mesh, 40-63 µm) from Sigma-Aldrich.

### Analytical Methods

¹H and ¹³C Nuclear magnetic resonance (NMR) spectroscopy was carried out using a 500 MHz Bruker Avance III spectrometer in the stated solvent at around room temperature unless otherwise stated. In all cases, NMR data were consistent with the proposed structures. Characteristic chemical shifts (δ) are given in parts-per-million using conventional abbreviations for designation of major peaks: e.g. s, singlet; d, doublet; t, triplet; q, quartet; dd, doublet of doublets; br, broad. Thin layer chromatography was run on Merck TLC Silica gel 60 F254 plates; typically 5 cm x 10 cm. Detection was achieved using a 254 nm UV source or permanganate stain.

### Compound Preparation

Where the preparation of starting materials is not described, these are commercially available, known in the literature, or readily obtainable by those skilled in the art using standard procedures. Where it is stated that compounds were prepared analogously to earlier examples or intermediates, it will be appreciated by the skilled person that the reaction time, number of equivalents of reagents and temperature can be modified for each specific reaction and that it may be necessary or desirable to employ different work-up or purification techniques. Where reactions were carried out using microwave irradiation, the microwave used was an Initiator 60 supplied by Biotage. The actual power supplied varies during the course of the reaction in order to maintain a constant temperature.

The synthesis of selected compound of the disclosure is described according to Scheme 1 and Scheme 2.

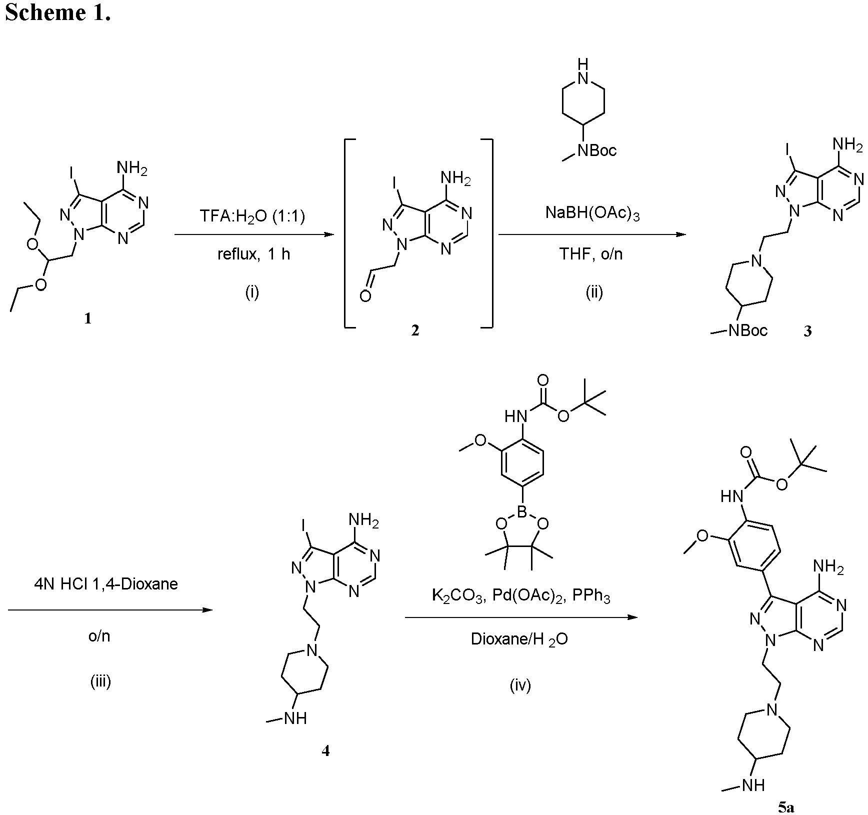

1-(2,2-Diethoxyethyl)-3-iodo-pyrazolo[3,4-d]pyrimidin-4-amine (1) was synthesized as previously reported. REF: Fraser, et al. Rapid Discovery and Structure-Activity Relationships of Pyrazolopyrimidines That Potently Suppress Breast Cancer Cell Growth via SRC Kinase Inhibition with Exceptional Selectivity over ABL Kinase. Journal of Medicinal Chemistry 2016, 59, 4697-4710.

### Synthesis of 1-[2-[4-(N-Boc-N-methylamino)-1-piperidyl]ethyl]-3-iodo-pyrazolo[3,4-d]pyrimidin-4-amine (3)

Compound **1** (500 mg, 1.33 mmol, 1.0 eq.) was added to a 20 mL microwave tube. H₂O:TFA (1:1) (15 mL) was added, and the mixture heated to 100 °C for 1h to obtain the aldehyde intermediate (compound **2**). The mixture was concentrated in vacuo to give a white solid which was used without further purification. The aldehyde was suspended in THF (16 mL). Tert-Butyl methyl(piperidin-4-yl)carbamate (500 mg, 1.59 mmol, 1.2 eq.) was added followed by a drop of TFA and the mixture was allowed to stir for 10 min. Sodium triacetoxyborohydride (281 mg, 1.33 mmol, 1.0 eq.) was added, and the mixture allowed to stir overnight. The reaction was quenched with H₂O (10 mL). EtOAc (10 mL) was added and the aqueous layer was adjusted to basic pH with sat. sodium bicarbonate solution. The aqueous layer was extracted with EtOAc (10 mL × 3). The organic layers were combined and dried with MgSO₄. The solvent was removed in vacuo and the crude was purified with flash chromatography (DCM:MeOH 85:15) to give the title compound as a brown solid (131 mg, 0.26 mmol, yield 20%).

¹H NMR (500 MHz, MeOD) δ 8.20 (s, 1H), 4.47 (t, *J* = 6.6 Hz, 2H), 3.11 - 3.02 (m, 3H), 2.87 (t, *J =* 6.6 Hz, 2H), 2.69 (s, 3H), 2.14 (t, *J =* 10.9 Hz, 2H), 1.65 (qd, *J =* 12.2, 3.9 Hz, 2H), 1.55 (d, *J=* 12.3 Hz, 2H), 1.44 (s, 9H). ¹

³C NMR (126 MHz, MeOD) δ 159.45, 157.35, 157.03, 155.05, 105.05, 88.32, 81.01, 57.67, 54.01, 45.77, 29.87, 28.86, 28.71.

### Synthesis of 1-[2-[4-(methylamino)-1-piperidyl]ethyl]-3-iodo-pyrazolo[3, 4-d]pyrimidin-4-amine (4)

Compound **3** (137 mg, 0.28 mmol, 1.0 eq.) was dissolved in Dioxane/HCl 4N (1 mL). The solution was stirred overnight at r.t. The solvent was removed *in vacuo.* Cation exchange beads and DCM/MeOH (8:2) (2 mL) was added and the solution was stirred gently overnight at r.t. The solution was filtered and the solvent was removed *in vacuo* to give the title compound as a brown solid (89 mg, 0.22 mmol, 80%).

¹H NMR (500 MHz, DMSO) δ 8.20 (s, 1H), 4.38 (t, *J =* 6.6 Hz, 2H), 2.96 (d, *J =* 10.7 Hz, 2H), 2.92 - 2.80 (m, 1H), 2.74 (s, 2H), 2.46 (s, 3H), 2.06 - 1.86 (m, 4H), 1.40 (d, *J =* 11.4 Hz, 2H).

¹³C NMR (126 MHz, DMSO) δ 157.65, 155.92, 153.59, 103.00, 88.99, 55.88, 54.99, 50.75, 45.31, 29.15, 27.54.

### Synthesis of tert-butyl (4-(4-amino-1-(2-(4-(methylamino)piperidin-1-yl)ethyl)-1H-pyrazolo[3,4-d]pyrimidin-3-yl)-2-methoxyphenyl)carbamate (5a)

Compound **4** (68 mg, 0.17 mmol, 1.0 eq.), 4-N-Boc-amino-3-methoxyphenylboronic acid pinacol ester (89 mg, 0.25 mmol, 1.5 eq.), K₂CO₃ (35 mg, 0.25 mmol, 1.5 eq.), PPh₃ (9 mg, 0.03 mmol, 0.2 equiv) and Pd(OAc)₂ (2 mg, 0.008 mmol, 0.05 eq.) were added to a 20 mL microwave tube. Dioxane:water (9:1) (13 mL) was added and the solution was heated in the microwave at 120 °C for 1 h. EtOAc (100 mL) and water (100 mL) were added to the mixture, and the organic layer separated. The aqueous layer was washed with EtOAc (50 mL, X2), and the organics combined, dried over anhydrous MgSO4, and concentrated in vacuo. The crude was purified via flash chromatography to yield the title compound as a brown solid (20 mg, 0.04 mmol, 24%).

¹H NMR (500 MHz, MeOD) δ 8.24 (s, 1H), 8.06 (d, *J* = 8.2 Hz, 1H), 7.27 (d, *J* = 1.8 Hz, 1H), 7.23 (dd, *J =* 8.2, 1.9 Hz, 1H), 4.53 (t, *J =* 6.5 Hz, 2H), 3.96 (s, 3H), 3.11 (d, *J =* 12.4 Hz, 2H), 2.97 - 2.85 (m, 3H), 2.61 (s, 3H), 2.17 (td, *J* = 12.0, 2.3 Hz, 2H), 2.04 - 1.96 (m, 2H), 1.90 (s, 3H), 1.58 - 1.40 (m, 11).

¹³C NMR (126 MHz, MeOD) δ 159.92, 156.81, 155.58, 154.84, 150.64, 146.39, 130.20, 128.75, 121.83, 120.95, 111.67, 99.08, 81.61, 57.41, 57.31, 56.50, 52.50, 45.47, 30.71, 29.65, 28.62.

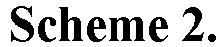

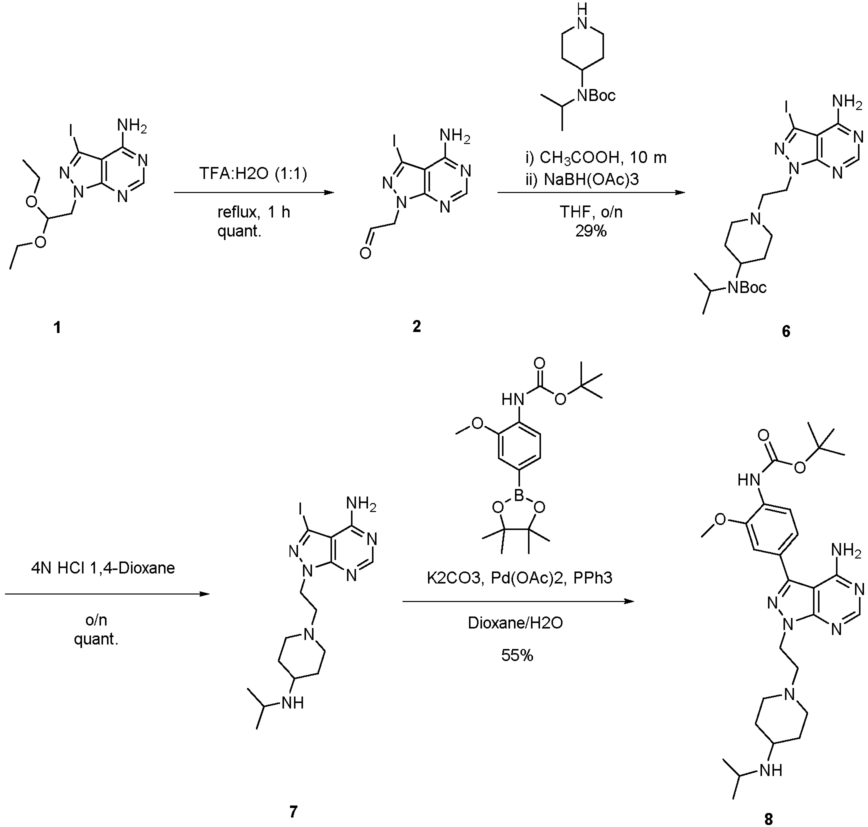

### Synthesis of Compound 6

Compound **1** (300 mg, 0.8 mmol, 1.0 eq.) was added to a 20 mL microwave tube. H₂O:TFA (1:1) (15 mL) was added, and the mixture heated to 100 °C for 1h to obtain the aldehyde intermediate (compound **2**). The mixture was concentrated in vacuo to give a white solid which was used without further purification. The aldehyde was suspended in THF (6 mL). *Tert*-butyl N-(piperidin-4-yl)-N-(propan-2-yl)carbamate (289 mg, 1.2 mmol, 1.5 eq.) was added followed by a drop of acetic acid and the mixture was allowed to stir for 10 min. Sodium triacetoxyborohydride (254 mg, 1.2 mmol, 1.5 eq.) was added, and the mixture allowed to stir overnight. The reaction was quenched with H₂O (10 mL). EtOAc (10 mL) was added and the aqueous layer was adjusted to basic pH with sat. sodium bicarbonate solution. The aqueous layer was extracted with EtOAc (10 mL × 3). The organic layers were combined and dried with MgSO₄. The solvent was removed in vacuo and the crude was purified with flash chromatography (DCM:MeOH 85:15) to give the title compound as a brown solid (121 mg, 0.23 mmol, yield 29%).

¹H NMR (500 MHz, CDCl₃) δ 8.31 (s, 1H), 6.07 (s, 2H), 4.46 (t, *J =* 6.9 Hz, 2H), 3.01 - 2.96 (m, 2H), 2.84 (t, *J =* 6.9 Hz, 2H), 2.10 (t, *J =* 11.3 Hz, 2H), 1.87 - 1.76 (m, 3H), 1.53 - 1.41 (m, 12H), 1.18 (d, *J =* 6.9 Hz, 6H).

¹³C NMR (126 MHz, CDCl₃) δ 157.54, 156.14, 155.62, 154.19, 104.09, 86.00, 79.44, 56.89, 53.62, 53.15, 46.16, 45.47, 30.11, 28.79, 21.66.

### Synthesis of Compound 8

Compound **3** (100 mg, 0.19 mmol, 1.0 eq.) was dissolved in dioxane/HCl 4N (1 mL). The solution was stirred overnight at r.t. The solvent was removed *in vacuo.* Cation exchange beads and DCM/MeOH (8:2) (2 mL) were added and the solution was stirred gently overnight at room temperature. The solution was filtered and compound 7 was obtained upon evaporation and used without further purification. Compound 7 (80 mg, 0.19 mmol, 1.0 eq.), (Boc)NH-bpin (98 mg, 0.27 mmol, 1.5 eq.), K₂CO₃ (47 mg, 0.34 mmol, 2.0 eq.), PPh₃ (9 mg, 0.03 mmol, 0.2 equiv) and Pd(OAc)₂ (2 mg, 0.008 mmol, 0.05 eq.) were added to a 20 mL microwave tube. Dioxane:water (9:1) (2.2 mL) was added and the solution was heated in the microwave at 120 °C for 1 h. EtOAc (100 mL) and water (100 mL) were added to the mixture, and the organic layer separated. The aqueous layer was washed with EtOAc (50 mL, X2), and the organics combined, dried over anhydrous MgSO4, and concentrated in vacuo. The crude was purified via flash chromatography to yield the title compound as a brown solid (55 mg, 0.1 mmol, 55%).

¹H NMR (500 MHz, MeOD) δ 8.24 (s, 1H), 8.05 (d, *J* = 8.2 Hz, 1H), 7.27 (d, *J* = 1.8 Hz, 1H), 7.22 (dd, *J* = 8.2, 1.8 Hz, 1H), 4.53 (t, *J =* 6.8 Hz, 2H), 3.95 (s, 3H), 3.08 - 2.97 (m, 3H), 2.90 (t, *J* = 6.8 Hz, 2H), 2.61 (tt, *J* = 11.0, 4.1 Hz, 1H), 2.13 (td, *J* = 12.0, 2.4 Hz, 2H), 1.87 (d, *J =* 12.3 Hz, 2H), 1.54 (s, 9H), 1.31 (qd, *J =* 12.3, 3.8 Hz, 2H), 1.06 (d, *J =* 6.3 Hz, 6H).

¹³C NMR (126 MHz, MeOD) δ 159.89, 156.77, 155.45, 154.81, 150.58, 146.36, 130.15, 128.76, 121.83, 120.88, 111.67, 99.12, 81.58, 57.81, 56.49, 53.58, 52.53, 45.70, 45.38, 32.64, 28.63, 22.35.

## Claims

1. A compound of formula I, or a pharmaceutically acceptable salt thereof, 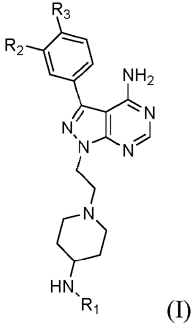 wherein:
R₁ is selected from a group consisting of alkyl, cycloalkyl, alkenyl, cycloalkenyl and alkynyl;
R₂ is selected from a group consisting of H, halo, OR₁₁, NHR₁₁, alkyl, alkenyl and alkynyl;
R₃ is selected from a group consisting of alkyl, alkenyl, alkynyl, aryl, halo, aryloxy, NHCO₂R₄, NHCONR₅R₆, NHCOR₇, NH-alkyl, NH-alkenyl, NH(CH₂)ₙ-aryl, (CH₂)ₚ-heteroaryl, (CH₂)_{q}CO₂R₈, (CH₂)ᵣCOR₉ and NHSO₂R₁₀, wherein each alkyl, alkenyl, aryl or heteroaryl moiety in the aforementioned list is optionally further substituted by one or more groups selected from alkyl, halo, OH, NH₂, alkoxy, aryloxy, alkylamino, arylamino, carboxyl and carboxamide;
R₄ to R₁₁ are each independently selected from a group consisting of alkyl, alkenyl and aryl; and
n is selected from a group consisting of 0, 1, 2, 3, 4, 5 and 6,
p is selected from a group consisting of 0, 1, 2, 3, 4, 5 and 6,
q is selected from a group consisting of 0, 1, 2, 3, 4, 5 and 6, and
r is selected from a group consisting of 0, 1, 2, 3, 4, 5 and 6.

2. The compound of claim 1, wherein R₁ is alkyl or alkenyl.

3. The compound of claim 1 or 2, wherein R₁ is CH₃.

4. The compound of claim 1 or 2, wherein R₁ is CH(CH₃)₂.

5. The compound of any one of claims 1-4, wherein R₂ is selected from a group consisting of H and alkoxy, preferably from H and OCH₃.

6. The compound of any one of claims 1-5, wherein R₄ to R₁₁ are each independently alkyl.

7. The compound of any one of claims 1-6, wherein R₃ is selected from a group consisting of NHCO₂-alkyl, NHCO-alkyl, NH(CH₂)ₙ-aryl, NHCONH-alkyl, (CH₂)ₚ-heteroaryl and (CH₂)_{q}CO₂-alkyl.

8. The compound of any one of claims 1-6, wherein R₃ is selected from a group consisting of NHCO₂-^{t}Bu, NHCOCH₂C(CH₃)₃, NHCH₂phenyl, NHCONH-^{t}Bu, CH₂-(4-methyl-oxazol-2-yl) and CH₂CO₂-^{t}Bu.

9. A compound of formula I which structure is: 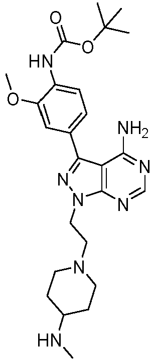 or a pharmaceutically acceptable salt thereof.

10. A compound of formula I which structure is: 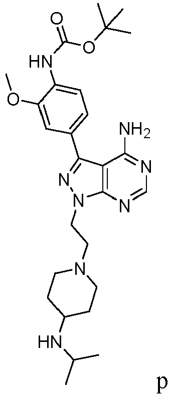 or a pharmaceutically acceptable salt thereof.

11. A combination comprising the compound of any one of claims 1-10, and a further therapeutic agent.

12. A pharmaceutical composition comprising the compound of any one of claims 1-10 or the combination of claim 11, and a pharmaceutically acceptable carrier, diluent or excipient.

13. A compound of any one of claims 1-10 for use in medicine.

14. A compound of any one of claims 1-10 for use in treating a mammal having a disease state alleviated by the selective inhibition of a SRC family kinase.

15. A compound of any one of claims 1-10 for use in treating cancer; or a viral infection, or osteoporosis; or a neurological disorder; or angiogenesis; or a lung disease; or metastatic bone disease; or atherosclerosis; or restenosis.

## Patentansprüche

1. Eine Verbindung der Formel I oder ein pharmazeutisch akzeptables Salz davon, 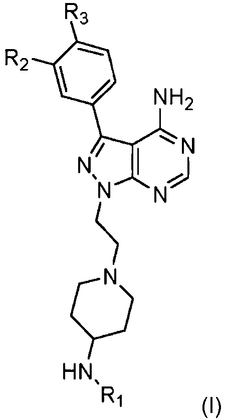 wobei:
R₁ aus einer Gruppe ausgewählt ist, die aus Alkyl, Cycloalkyl, Alkenyl, Cycloalkenyl und Alkinyl besteht;
R₂ aus einer Gruppe ausgewählt ist, die aus H, Halogen, OR₁₁, NHR₁₁, Alkyl, Alkenyl und Alkinyl besteht;
R₃ aus einer Gruppe ausgewählt ist, die aus Alkyl, Alkenyl, Alkinyl, Aryl, Halogen, Aryloxy, NHCO₂R₄, NHCONR₅R₆, NHCOR₇, NH-Alkyl, NH-Alkenyl, NH(CH₂)ₙ-Aryl, (CH₂)ₚ-Heteroaryl, (CH₂)_{q}CO₂R₈, (CH₂)ᵣCOR₉ und NHSO₂R₁₀ besteht, wobei jeder Alkyl-, Alkenyl-, Aryl- oder Heteroarylmolekülteil in der oben genannten Liste optional ferner mit einer oder mehreren Gruppen substituiert ist, die aus Alkyl, Halogen, OH, NH₂, Alkoxy, Aryloxy, Alkylamino, Arylamino, Carboxyl und Carboxamid ausgewählt sind;
R₄ bis R₁₁ jeweils unabhängig aus einer Gruppe ausgewählt sind, die aus Alkyl, Alkenyl und Aryl besteht; und
n aus einer Gruppe ausgewählt ist, die aus 0, 1, 2, 3, 4, 5 und 6 besteht,
p aus einer Gruppe ausgewählt ist, die aus 0, 1, 2, 3, 4, 5 und 6 besteht,
q aus einer Gruppe ausgewählt ist, die aus 0, 1, 2, 3, 4, 5 und 6 besteht, und
r aus einer Gruppe ausgewählt ist, die aus 0, 1, 2, 3, 4, 5 und 6 besteht.

2. Verbindung gemäß Anspruch 1, wobei R₁ Alkyl oder Alkenyl ist.

3. Verbindung gemäß Anspruch 1 oder 2, wobei R₁ CH₃ ist.

4. Verbindung gemäß Anspruch 1 oder 2, wobei R₁ CH(CH₃)₂ ist.

5. Verbindung gemäß einem der Ansprüche 1-4, wobei R₂ aus einer Gruppe ausgewählt ist, die aus H und Alkoxy, vorzugsweise aus H und OCH₃, besteht.

6. Verbindung gemäß einem der Ansprüche 1-5, wobei R₄ bis R₁₁ jeweils unabhängig Alkyl sind.

7. Verbindung gemäß einem der Ansprüche 1-6, wobei R₃ aus einer Gruppe ausgewählt ist, die aus NHCO₂-Alkyl, NHCO-Alkyl, NH(CH₂)ₙ-Aryl, NHCONH-Alkyl, (CH₂)ₚ-Heteroaryl und (CH₂)₄CO₂-Alkyl besteht.

8. Verbindung gemäß einem der Ansprüche 1-6, wobei R₃ aus einer Gruppe ausgewählt ist, die aus NHCO₂-^{t}Bu, NHCOCH₂C(CH₃)₃, NHCH₂-Phenyl, NHCONH-^{t}Bu, CH₂-(4-Methyloxazol-2-yl) und CH₂CO₂-^{t}Bu besteht.

9. Eine Verbindung der Formel I, wobei die Struktur wie folgt ist: 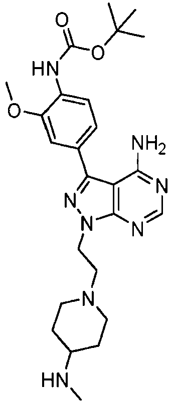 oder ein pharmazeutisch akzeptables Salz davon.

10. Eine Verbindung der Formel I, wobei die Struktur wie folgt ist: 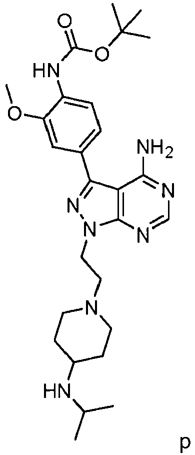 oder ein pharmazeutisch akzeptables Salz davon.

11. Eine Kombination, die die Verbindung gemäß einem der Ansprüche 1-10 und ein weiteres Therapeutikum beinhaltet.

12. Eine pharmazeutische Zusammensetzung, die die Verbindung gemäß einem der Ansprüche 1-10 oder die Kombination gemäß Anspruch 11 und einen pharmazeutisch akzeptablen Träger, ein pharmazeutisch akzeptables Verdünnungsmittel oder einen pharmazeutisch akzeptablen Hilfsstoff beinhaltet.

13. Verbindung gemäß einem der Ansprüche 1-10 zur Verwendung in der Medizin.

14. Verbindung gemäß einem der Ansprüche 1-10 zur Verwendung bei der Behandlung eines Säugetiers, das einen Krankheitszustand aufweist, der durch die selektive Hemmung einer Kinase der SRC-Familie gelindert wird.

15. Verbindung gemäß einem der Ansprüche 1-10 zur Verwendung bei der Behandlung von Krebs oder einer Virusinfektion oder Osteoporose oder einer neurologischen Störung oder Angiogenese oder einer Lungenerkrankung oder einer metastatischen Knochenerkrankung oder Atherosklerose oder Restenose.

## Revendications

1. Un composé de formule I, ou un sel pharmaceutiquement acceptable de celui-ci, 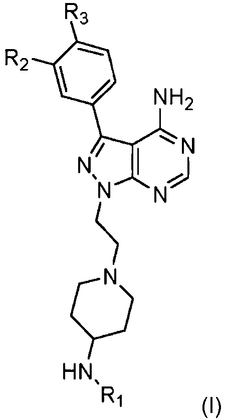 dans lequel :
R₁ est sélectionné dans un groupe constitué d'un alkyle, d'un cycloalkyle, d'un alcényle, d'un cycloalcényle et d'un alcynyle ;
R₂ est sélectionné dans un groupe constitué de H, d'un halo, de OR₁₁, de NHR₁₁, d'un alkyle, d'un alcényle et d'un alcynyle ;
R₃ est sélectionné dans un groupe constitué d'un alkyle, d'un alcényle, d'un alcynyle, d'un aryle, d'un halo, d'un aryloxy, de NHCO₂R₄, de NHCONR₅R₆, de NHCOR₇, de NH-alkyle, de NH-alcényle, de NH(CH₂)ₙ-aryle, de (CH₂)ₚ-hétéroaryle, de (CH₂)_{q}CO₂R₈, de (CH₂)ᵣCOR₉ et de NHSO₂R₁₀, dans lequel chaque partie alkyle, alcényle, aryle ou hétéroaryle dans la liste susmentionnée est optionnellement en outre substituée par un ou plusieurs groupes sélectionnés parmi un alkyle, un halo, OH, NH₂, un alcoxy, un aryloxy, un alkylamino, un arylamino, un carboxyle et un carboxamide ;
R₄ à R₁₁ sont chacun indépendamment sélectionnés dans un groupe constitué d'un alkyle, d'un alcényle et d'un aryle ; et
n est sélectionné dans un groupe constitué de 0, 1, 2, 3, 4, 5 et 6,
p est sélectionné dans un groupe constitué de 0, 1, 2, 3, 4, 5 et 6,
q est sélectionné dans un groupe constitué de 0, 1, 2, 3, 4, 5 et 6, et
r est sélectionné dans un groupe constitué de 0, 1, 2, 3, 4, 5 et 6.

2. Le composé de la revendication 1, dans lequel R₁ est un alkyle ou un alcényle.

3. Le composé de la revendication 1 ou la revendication 2, dans lequel R₁ est CH₃.

4. Le composé de la revendication 1 ou la revendication 2, dans lequel R₁ est CH(CH₃)₂.

5. Le composé de l'une quelconque des revendications 1 à 4, dans lequel R₂ est sélectionné dans un groupe constitué de H et d'un alcoxy, de préférence parmi H et OCH₃.

6. Le composé de l'une quelconque des revendications 1 à 5, dans lequel R₄ à R₁₁ sont chacun indépendamment un alkyle.

7. Le composé de l'une quelconque des revendications 1 à 6, dans lequel R₃ est sélectionné dans un groupe constitué de NHCO₂-alkyle, NHCO-alkyle, NH(CH₂)ₙ-aryle, NHCONH-alkyle, (CH₂)ₚ-hétéroaryle et (CH2)_{q}CO₂-alkyle.

8. Le composé de l'une quelconque des revendications 1 à 6, dans lequel R₃ est sélectionné dans un groupe constitué de NHCO₂-^{t}Bu, NHCOCH₂C(CH₃)₃, NHCH₂phényle, NHCONH-^{t}Bu, CH₂-(4-méthyl-oxazol-2-yle) et CH₂CO₂-^{t}Bu.

9. Un composé de formule I dont la structure est : 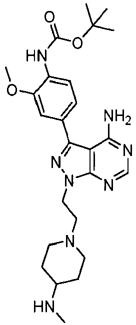 ou un sel pharmaceutiquement acceptable de celui-ci.

10. Un composé de formule I dont la structure est : 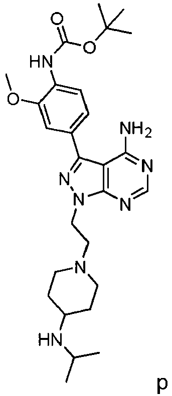 ou un sel pharmaceutiquement acceptable de celui-ci.

11. Une combinaison comprenant le composé de l'une quelconque des revendications 1 à 10, et un autre agent thérapeutique.

12. Une composition pharmaceutique comprenant le composé de l'une quelconque des revendications 1 à 10 ou la combinaison de la revendication 11, et un support, diluant ou excipient pharmaceutiquement acceptable.

13. Un composé de l'une quelconque des revendications 1 à 10 pour son utilisation en médecine.

14. Un composé de l'une quelconque des revendications 1 à 10 pour son utilisation dans le traitement d'un mammifère ayant un état de maladie soulagé par l'inhibition sélective d'une kinase de la famille SRC.

15. Un composé de l'une quelconque des revendications 1 à 10 pour son utilisation dans le traitement du cancer ; ou d'une infection virale, ou de l'ostéoporose ; ou d'un trouble neurologique ; ou de l'angiogenèse ; ou d'une maladie pulmonaire ; ou d'une maladie osseuse métastatique ; ou de l'athérosclérose ; ou de la resténose.
